(19) Europäisches Patentamt
European Patent Office
Office européen des brevets

(11) Veröffentlichungsnummer : **0 129 759**
**B1**

(12) # EUROPÄISCHE PATENTSCHRIFT

(45) Veröffentlichungstag der Patentschrift : **06.05.87**

(51) Int. Cl.⁴ : **C 07 C125/06**

(21) Anmeldenummer : **84106632.7**

(22) Anmeldetag : **09.06.84**

(54) **Verfahren zur Herstellung von Urethanen.**

(30) Priorität : **23.06.83 DE 3322668**

(43) Veröffentlichungstag der Anmeldung : **02.01.85 Patentblatt 85/01**

(45) Bekanntmachung des Hinweises auf die Patenterteilung : **06.05.87 Patentblatt 87/19**

(84) Benannte Vertragsstaaten : **BE DE FR GB IT NL**

(56) Entgegenhaltungen :
**EP-A- 0 035 752**
**DD-A- 105 211**
**DE-A- 2 819 826**
**DE-A- 2 903 950**
**PATENT ABSTRACTS OF JAPAN, Band 3, Nr. 138, 16. November 1979, Seite 18 C 64;**
**PATENT ABSTRACTS OF JAPAN, Band 4, Nr. 34, 22. März 1980, Seite (C-3) (516);**

(73) Patentinhaber : **BAYER AG**
**Konzernverwaltung RP Patentabteilung**
**D-5090 Leverkusen 1 Bayerwerk (DE)**

(72) Erfinder : **Stammann, Günter, Dr.**
**Silesiusstrasse 78**
**D-5000 Köln 80 (DE)**
Erfinder : **Grolig, Johann, Dr.**
**Heinrich-Luebke-Strasse 22**
**D-5090 Leverkusen 1 (DE)**
Erfinder : **Becker, Robert, Dr.**
**Martin-Heidegger-Strasse 8**
**D-5090 Leverkusen 3 (DE)**
Erfinder : **Waldmann, Helmut, Dr.**
**Henry T. von Boettinger-Strasse 15**
**D-5090 Leverkusen 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft ein verbessertes Verfahren zur Herstellung von Urethanen (Carbamidsäureestern), bei welchem organische Nitroverbindungen und mindestens eine Hydroxygruppe enthaltende organische Verbindungen in einer Hydrocarbonylierungsreaktion mit einem sowohl Kohlenmonoxid als auch in erheblicher Konzentration Wasserstoff enthaltendem Reaktionsgas in Gegenwart der Edelmetalle aus der 8. Nebengruppe des Periodensystems und bestimmten Cokatalysatoren umgesetzt werden.

Die Herstellung von Urethanen aus organischen Nitroverbindungen, organischen Hydroxylverbindungen und Kohlenmonoxid in Gegenwart von Edelmetall-Katalysatoren ist bereits bekannt. So beschreiben beispielsweise die deutschen Offenlegungsschriften 28 19 826 und 29 03 950 derartige Verfahren, die wegen der Mitverwendung spezieller Cokatalysatoren wie Eisenoxichlorid oder Kombinationen von Eisenoxiden bzw. Eisenoxidhydraten mit aktivierenden Chloriden nebst tert. Aminen die Herstellung der Urethane in sehr guten Raum/Zeit-Ausbeuten gestatten. Obwohl die in diesen beispielhaft genannten Vorveröffentlichungen beschriebene Urethansynthese die Möglichkeit der phosgenfreien Isocyanatherstellung durch thermische Spaltung der Urethane gestattet, fand bislang keines dieser Verfahren Eingang in die großtechnische Praxis. Einer der Gründe hierfür dürfte in der noch nicht optimalen Kostenstruktur der Verfahren liegen.

Es war daher die der vorliegenden Erfindung zugrundeliegende Aufgabe, die bekannten Verfahren zur Herstellung von Urethanen aus den genannten Ausgangsmaterialien so zu verbessern, daß sie kostengünstiger durchführbar sind.

Überraschenderweise konnte diese Aufgabe dadurch gelöst werden, daß die Nitroverbindungen und organischen Hydroxylverbindungen nicht mit reinem Kohlenoxid, sondern mit einem Gemisch aus Kohlenoxid und Wasserstoff in Gegenwart bestimmter Katalysatorsysteme in einer Hydrocarbonylierungsreaktion zur Umsetzung gebracht werden.

Die unter der Nummer 55-7227 (1980) veröffentlichte japanische Patentanmeldung (Anmeldung 53/79076 v. 29.6.1978) beschreibt zwar bereits, daß die Urethanbildung aus den genannten Ausgangsmaterialien in Gegenwart von Palladiumkatalysatoren durch die Gegenwart geringer Wasserstoffmengen beschleunigt wird, hebt jedoch hervor, daß bei mehr als 6 Vol.-% Wasserstoff im Reaktionsgas die Urethanausbeute sinkt.

Im übrigen scheint das Verfahren der Vorveröffentlichung, wie aus den Beispielen ersichtlich, auf die Mitverwendung von Selen als wesentlicher Katalysatorkomponente angewiesen zu sein. Beim nachstehend näher beschriebenen erfindungsgemäßen Verfahren, welches in Abwesenheit von Selen durchgeführt wird, ist jedoch Wasserstoff in erheblichen Mengen zugegen und tritt, wie weiter unten ausgeführt, stöchiometrisch in die Urethanbildungsreaktion ein. Hiermit konnte aufgrund der Lehre der genannten Vorveröffentlichung, die sich nicht der beim erfindungsgemäßen einzusetzenden Cokatalysatoren bedient, nicht gerechnet werden.

Die unter der Nummer 57-185253 im Jahre 1982 veröffentlichte japanische Patentanmeldung (Anmeldung 56-68249 v. 8.5.1981) beschreibt den Einsatz von Gemischen aus Kohlenoxid und Wasserstoff bei der Urethansynthese aus aromatischen Nitroverbindungen unter gleichzeitiger Mitverwendung von, den Nitroverbindungen entsprechenden Aminoverbindungen, die gleichzeitig zu Urethanen umgesetzt werden. Bei diesem Verfahren, bei welchem ebenfalls die erfindungsgemäß einzusetzenden Cokatalysatoren nicht mitverwendet werden, müssen im übrigen, ausweislich der allgemeinen Beschreibung und der Ausführungsbeispiele, unwirtschaftlich hohe Mengen an Edelmetallkatalysatoren eingesetzt werden.

Gegenstand der Erfindung ist ein Verfahren zur Herstellung von Urethanen durch Umsetzung von organischen Nitroverbindungen mit mindestens eine Hydroxygruppe enthaltenden organischen Verbindungen und Kohlenoxid in Gegenwart eines Selen-freien Katalysatorsystems, welches

a) mindestens ein Edelmetall oder eine Edelmetallverbindung der 8. Nebengruppe des Periodensystems der Elemente enthält, dadurch gekennzeichnet, daß man einerseits ein solches Katalysatorsystem verwendet, welches.

b) mindestens einen Cokatalysator bestehend aus einer Kombination aus

ba) mindestens einer oxidischen oder hydroxidischen Verbindung des Eisens oder des Kupfers und

bb) mindestens einer, anionisch als Chlorid gebundenes Chlor enthaltenden Verbindung ausgewählt aus der Gruppe bestehend aus

(i) Chloriden oder Oxichloriden von Elementen der dritten bis fünften Hauptgruppe und/oder der ersten bis achten Nebengruppe des Periodensystems mit Ausnahme der Edelmetalle der achten Nebengruppe und

(ii) organischen Ammoniumchloriden, und

c) als weitere Cokatalysatorkomponente mindestens eine organische Stickstoffbase

enthält, und man andererseits das Kohlenoxid zusammen mit Wasserstoff unter Einhaltung eines Molverhältnisses CO : $H_2$ von 0,3 : 1 bis 3 : 1 verwendet.

Bei den bisher üblichen Verfahren zur Herstellung von Urethanen aus Nitroverbindungen, organischen Hydroxyverbindungen und Kohlenoxid werden die Nitroverbindungen unter Verbrauch von insgesamt 3 Molekülen Kohlenmonoxid pro Nitrogruppe reduziert und carbonyliert, wie dies beispielsweise aus Gleichung (1) hervorgeht :

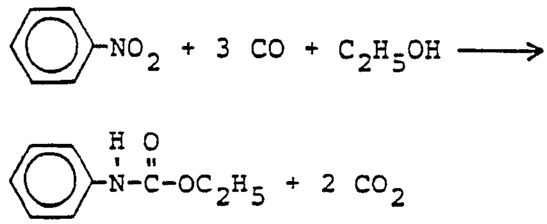

(1)

Dem erfindungsgemäßen Verfahren liegt als wesentliche Reaktion die beispielsweise nach Gleichung (2) ablaufende Hydrocarbonylierung unter Verbrauch von nur einem Molekül CO je umzusetzende Nitrogruppe zugrunde.

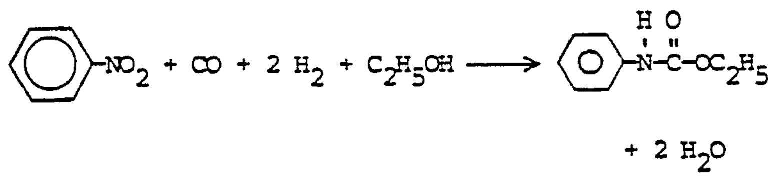

(2)

Die nach Gleichung (1) ablaufende unökonomischere Carbonylierung unter Verbrauch von 3 Molekülen CO je Nitrogruppe findet je nach Reaktionsbedingungen ebenfalls in gewissem Umfang statt, so daß sich als Gesamtreaktion die in Gleichung (3) ausgedrückte Stöchiometrie ergibt, formuliert am Beispiel Phenylurethan.

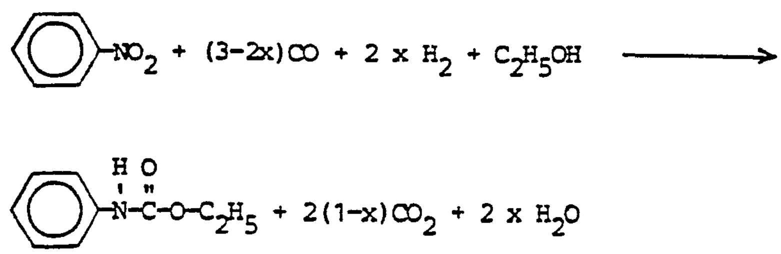

(3)

Die Gleichungen (1) und (2) sowie die daraus abgleitete Gleichung (3) sollen nicht dazu dienen, den Reaktionsablauf in irgendeiner Weise theoretisch zu deuten, sie erfassen nur summarisch die Einsatzstoffe und die Hauptprodukte des erfindungsgemäßen Verfahrens am Beispiel der Herstellung von N-Phenyl-O-ethyl-urethan.

Der wesentliche Vorteil des erfindungsgemäßen Verfahrens ist darin zu sehen, daß anstelle von Kohlenmonoxid Gemische aus Kohlenmonoxid und Wasserstoff als Reaktionsgas eingesetzt werden können. Im allgemeinen sind Kohlenmonoxid/Wasserstoffgemische, wenn man CO und $H_2$ als gleichwertige Reduktionsäquivalente rechnet, billiger als reines Kohlenmonoxid. Es ist einleuchtend, daß ein Verfahren, welches als Einsatzgas Gemische von Kohlenmonoxid und Wasserstoff verwenden kann, kostengünstiger ist als ein Verfahren, bei welchem reines Kohlenmonoxid eingesetzt werden muß, das erst aus einem derartigen Kohlenmonoxid/Wasserstoffgemisch, z. B. durch Tieftemperaturzerlegung oder nach dem Cosorb-Verfahren abgetrennt werden muß. In der Tat entfallen von den Herstellungskosten für Kohlenmonoxid aus einem Reformierungsverfahren ein erheblicher Anteil auf die Energien, die für die Trennung von Kohlenmonoxid und Wasserstoff aufzubringen sind und auf die damit verbundenen Investitionen.

Der besondere Vorteil des erfindungsgemäßen Verfahrens liegt in der chemischen Ausnutzung des im Einsatzgas vorhandenen Wasserstoffs für die Urethanbildung. Nach Gleichung (1) werden zwei Moleküle Kohlenmonoxid für die Reduktion der Nitrogruppe verwendet, welche als $CO_2$ verloren gehen und ein Molekül in das Urethanmolekül eingebaut. Beim erfindungsgemäßen Verfahren gemäß Gleichung (2) wird hingegen die Reduktion der Nitrogruppe durch zwei Wasserstoffmoleküle bewirkt, und ein Kohlenmonoxidmolekül dient zum Aufbau der Urethangruppe.

Es ist im übrigen überraschend, daß es erfindungsgemäß gelingt, die Urethanbildung weitgehend gemäß Gleichung (2) durchzuführen, obwohl in stöchiometrischen Mengen Wasser gebildet wird, da durchaus erwartet werden mußte, daß die gebildeten Urethane unter den Reaktionsbedingungen in Gegenwart des Reaktionswassers hydrolytisch zerstört werden.

Ausgangsverbindungen für das erfindungsgemäße Verfahren sind beliebige organische Nitroverbindungen, d. h. beliebige Nitrogruppen aufweisende, ansonsten unter den Bedingungen des erfindungsgemäßen Verfahrens inerte organische Verbindungen mit mindestens einer aliphatisch, cycloaliphatisch und/oder aromatisch gebundenen Nitrogruppe eines im allgemeinen zwischen 61 und 400, vorzugsweise 123 und 286 liegenden Molekulargewichts sowie beliebige, mindestens eine Hydroxygruppe enthaltende organische Verbindung, beispielsweise substituierte oder unsubstituierte aliphatische, cycloaliphatische und/oder aromatische Mono-, Di- oder Polyhydroxyverbindungen eines im allgemeinen zwischen 32 und 228, vorzugsweise zwischen 32 und 102 liegenden Molekulargewichts.

Beispielsweise können folgende aromatische Nitroverbindungen eingesetzt werden :

Nitrobenzol, o-Dinitrobenzol, m-Dinitrobenzol, p-Dinitrobenzol, o-Chlornitrobenzol, m-Chlor-nitrobenzol, o-Chlornitrobenzol, o-Nitrotoluol, m-Nitrotoluol, p-Nitrotoluol, 2,3-Dinitrotoluol, 2,4-Dinitrotoluol, 2,5-Dinitrotoluol, 2,6-Dinitrotoluol, 3,4-Dinitrotoluol, 3-Nitro-o-xylol, 4-Nitro-o-xylol, 2-Nitro-m-xylol, 4-Nitro-m-xylol, 5-Nitro-m-xylol, Nitro-p-xylol, 3,4-Dinitro-o-xylol, 3,5-Dinitro-o-xylol, 3,6-Dinitro-o-xylol, 4,8-Dinitro-o-xylol, 2,4-Dinitro-m-xylol, 2,5-Dinitro-m-xylol, 4,5-Dinitro-m-xylol, 4,6-Dinitro-m-xylol, 2,3-Dinitro-p-xylol, 2,6-Dinitro-p-xylol, 1-Nitronaphthalin, 2-Nitro-naphthalin, Dinitronaphthaline, Nitroanthracene, Nitrodiphenyle, Bis-(nitrophenyl)-methane, Bis-(nitrophenyl)-thioether, Bis-(nitrophenyl)-sulfone, Nitrodiphenoxyalkane, Nitrophenothiazine sowie Dinitroverbindungen gemäß EP-PS-0 024 665.

Als cycloaliphatische Nitroverbindungen seien genannt :

Nitrocyclobutan, Nitrocyclopentan, Nitrocyclohexan, 1,2-Dinitrocyclohexan, 1,3-Dinitrocyclohexan, 1,4-Dinitrocyclohexan, Bis-(nitrocyclohexyl)-methane.

Beispielhaft für die Gruppe der Nitroalkane seien genannt :

Nitromethan, Nitroethan, 1-Nitropropan, 2-Nitropropan, Nitrobutane, Nitropentane, Nitrohexane, Nitrodecane, Nitrocetane, 1,2-Dinitroethan, 1,2-Dinitropropan, 1,3-Dinitropropan, Dinitrobutane, Dinitropentane, Dinitrohexane, Dinitrodecane, Phenylnitromethan, Bis-(nitromethyl)-cyclohexane, Bis-(nitromethyl)-benzole, ω-Nitrocarbonsäurenitrile.

Bevorzugte Nitroverbindungen für das erfindungsgemäße Verfahren sind aromatische Nitroverbindungen wie insbesondere Nitrobenzol, 1,3-Dinitrobenzol, 2,4-Dinitrotoluol, 2,6-Dinitrotoluol, Dinitronaphthaline wie z. B. 1,5-Dinitronaphthalin oder 2,4'- bzw. 4,4'-Dinitrodiphenylmethan. Weitere besonders bevorzugte Nitroverbindungen für das erfindungsgemäße Verfahren sind zweikernige aromatische Dinitroverbindungen aus der Diphenylmethanreihe des Typs, der in der EP-PS-0 024 665 als Zwischenprodukt für Diisocyanate beschrieben ist. Besonders bevorzugt werden Nitrobenzol und technische Isomerengemische des Dinitrotoluols, welche 2,4-Dinitrotoluol als Hauptbestandteil enthalten, eingesetzt.

Zu den erfindungsgemäß geeigneten Hydroxygruppen enthaltenden organischen Verbindungen zählen einwertige Alkohole, mehrwertige Alkohole, einwertige Phenole und mehrwertige Phenole. Die Alkohole sind gegenüber den Phenolen bevorzugt.

Die Alkohole umfassen lineare oder verzweigte Alkanole, Cycloalkanole, Alkenole, Cycloalkenole, Aralkylalkohole und ähnliches, jeder ein- oder mehrwertig. Diese Alkohole können einen Substituenten enthalten, der Sauerstoff, Stickstoff, Schwefel oder ein Halogenatom enthält, beispielsweise eine Halogen-, Sulfoxid-, Sulfon-, Amin-, Amid-, Carbonyl- oder Carbonsäureestergruppe. Beispielhaft seien folgende einwertige Alkohole genannt : Methylalkohol, Ethylalkohol, Propanol, Isopropanol, Butanol, Pentanol, Hexanol, Cyclohexanol, Benzylalkohol. Geeignete mehrwertige Alkohole sind beispielsweise : Ethylenglykol, Propylenglykol, Dipropylenglykol, Glycerin, Hexantriol und ähnliche sowie höherfunktionelle Polyole. Bevorzugt werden einwertige aliphatische Alkohole mit 1 bis 6 Kohlenstoffatomen und besonders bevorzugt Ethylalkohol eingesetzt.

Zu den erfindungsgemäß geeigneten Phenolen zählen beispielsweise Phenol, Chlorphenole, Kresole, Ethylphenole, Propylphenole, Butylphenol oder höhere Alkylphenole, Brenzcatechin, Resorcin, 4,4'-Dihydroxydiphenylmethan, Bisphenol-A, Anthranol, Phenanthrol, Pyrogallol oder Phloroglucin.

Die organischen Hydroxyverbindungen werden bei der Durchführung des erfindungsgemäßen Verfahrens im allgemeinen in solchen Mengen eingesetzt, daß bei Verwendung von Mononitroverbindungen als Ausgangsmaterial ein Äquivalentverhältnis zwischen Nitrogruppen und Hydroxylgruppen von 1 : 0,5 bis 1 : 100, vorzugsweise 1 : 1 bis 1 : 100 und bei Verwendung von Dinitroverbindungen ein Äquivalentverhältnis zwischen Nitrogruppen und Hydroxylgruppen von 1 : 1 bis 1 : 100 vorliegt.

Besonders bevorzugt werden die bevorzugten Alkohole im Überschuß eingesetzt, wobei der nichtumgesetzte Überschuß als Reaktionsmedium dient.

Die beim erfindungsgemäßen Verfahren zum Einsatz kommenden Katalysatorsysteme enthalten neben der wesentlichen Edelmetallkomponente a) eine Cokatalysatorkomponente b), die eine zu Redoxreaktionen befähigte Metallverbindung enthält, sowie eine weitere Cokatalysatorkomponente c), die ein organisches Amin darstellt.

Bei dem Edelmetallkatalysator a) handelt es sich entweder um freie Edelmetalle der 8. Nebengruppe des Periodensystems oder um im Reaktionsgemisch lösliche Verbindungen dieser Metalle. Beispielsweise können die Edelmetalle in feinverteilter metallischer Form auf inerten Trägern wie z. B. Aktivkohle, Aluminiumoxid, Quarz, unlöslichen Silikaten, Bariumsulfat, Molybdänoxid, Wolframoxid oder unlöslichen spinellartigen Verbindungen eingesetzt werden. Vorteilhafter werden die Edelmetalle als im Reaktionsgemisch lösliche Verbindungen zugegeben, beispielsweise als Chloride, Bromide, Jodide, Chlorokomplexe, Bromokomplexe, Jodokomplexe, Acetate, Acetylacetonate u. a. lösliche Edelmetallverbindungen. Jedoch können auch an sich schlecht lösliche Edelmetallverbindungen, z. B. Oxide, eingesetzt werden, da löslichkeitsvermittelnde Cokatalysatorkomponenten, beispielsweise aktivierende Chloride, verwendet werden.

Geeignete Edelmetallkomponenten a) sind Ru, Rh, Pd, Os und Pt. Bevorzugte Edelmetalle sind Ru, Rh und Pd. Besonders bevorzugt werden Palladium und Ruthenium, insbesondere in Form der löslichen Chloride, eingesetzt.

Die bevorzugten Konzentrationen der Edelmetallkomponente a), bezogen auf das Reaktionsgemisch incl. gegebenenfalls mitverwendetem Lösungsmittel, liegen im allgemeinen bei 0,000 1 bis 0,1 Gew.-%, insbesondere bei 0,005 bis 0,05 Gew.-%, berechnet als Edelmetall. Bei niedrigeren Edelmetallkonzentrationen wird die Reaktionsgeschwindigkeit zu gering. Höhere Edelmetall-Konzentrationen sind zwar möglich, jedoch unwirtschaftlich, da ohnehin keine weitere Steigerung der Urethanausbeute erfolgt.

Die Cokatalysatorkomponente b) besteht aus einer Kombination aus ba) mindestens einer oxidischen oder hydroxidischen Verbindung des Eisens oder Kupfers mit bb) anionisch als Chlorid gebundenes Chlor enthaltenden Verbindungen ausgewählt aus der Gruppe bestehend aus (i) Chloriden oder Oxichloriden von Elementen der dritten bis fünften Hauptgruppe und/oder der ersten bis achten Nebengruppe des Periodensystems der Elemente mit Ausnahme der Edelmetalle der achten Nebengruppe und (ii) organischen Ammoniumchloriden.

Geeignete Cokatalysatorkomponenten ba) sind beispielsweise FeO, $\alpha$-$Fe_2O_3$, $\gamma$-$Fe_2O_3$, $\alpha$-FeO(OH), $\beta$-FeO(OH), $Fe(OH)_2$, $Fe(OH)_3$, $Cu_2O$, CuO, $Cu_2O(OH)_2$, $Cu(OH)_2$ oder $Cu_2(OH)_2CO_3$, sowie weitere Oxide, Hydroxide, Oxidhydrate oder Hydroxycarbonate (basische Carbonate) des Eisens oder des Kupfers. Bevorzugte Cokatalysatorkomponenten ba) sind die vorstehend formelmäßig bezeichneten Verbindungen, besonders bevorzugt sind $\alpha$- und $\gamma$-$Fe_2O_3$.

Die Cokatalysatorkomponenten bb) bestehen entweder aus (i) anionisch als Chlorid gebundenes Chlor enthaltenden Verbindungen von Elementen der dritten bis fünften Hauptgruppe und/oder der ersten bis achten Nebengruppe des Periodensystems der Elemente mit Ausnahme der Edelmetalle der achten Nebengruppe, die vorzugsweise, gegebenenfalls unter Hydrolyse oder Alkoholyse im Reaktionsgemisch zumindest teilweise löslich sind, oder aus (ii) organischen Ammoniumchloriden. Geeignete Verbindungen (i) sind beispielsweise $AlCl_3$, $TiOCl_2$, $VCl_3$, $VCl_5$, $VOCl_3$, $CrCl_3$ $MnCl_2$, MnOCl, $FeCl_2$, FeOCl, $FeCl_3$, $CoCl_2$, $NiCl_2$, $Cu_2Cl_2$, $Cu_2OCL_2$, $Cu_2OCl$, $ZnCl_2$, $NbCl_5$, $MoCl_6$, $WCl_6$. Die beispielhaft genannten Metallchloride können selbstverständlich auch in Form von Komplexsalzen beispielsweise in Form der entsprechenden Chloridhydrate oder Komplexen mit Aminbasen eingesetzt werden. Geeignete organische Ammoniumchloride (ii) sind beispielsweise Tetramethylammoniumchlorid, Tetraethylammoniumchlorid, Tetrabutylammoniumchlorid, beliebige quaternäre Ammoniumchloride, die unterschiedliche Alkylreste am Stickstoff tragen wie beispielsweise Methyl-tributylammoniumchlorid, und insbesondere Hydrochloride der nachstehend beispielhaft genannten als weitere Cokatalysatorkomponente eingesetzten Aminbasen c). Selbstverständlich ist es auch möglich als Komponente (ii) Verbindungen einzusetzen, die in situ zu Ammoniumchloriden der beispielhaft genannten Art abreagieren, beispielsweise ist die Verwendung von Carbamidsäurechloriden, wie sie durch Anlagerung von HCl an Isocyanate entstehen, ebenfalls möglich, da derartige Carbamidsäurechloride hydrolytisch in die entsprechenden Ammoniumchloride überführt werden. Bevorzugte Cokatalysatorkomponenten (i) sind $MnCl_2$, $FeCl_2$, $FeCl_3$ und $CuCl_2$ bzw. deren Hydrate oder Aminkomplexe, besonders bevorzugt sind die Eisenchloride bzw. deren Hydrate oder Aminkomplexe. Bevorzugte Cokatalysatorkomponenten (ii) sind die Hydrochloride der nachstehend beispielhaft genannten Amine c). Selbstverständlich ist es auch möglich beliebige Kombinationen der unter (i) bzw. (ii) beispielhaft genannten Verbindungen einzusetzen. Selbstverständlich ist es auch möglich, anstelle der genannten Kombinationen aus den Komponenten ba) und bb) Verbindungen einzusetzen, die eine stöchiometrische Kombination derartiger Einzelverbindungen darstellen. So ist es beispielsweise möglich anstelle der Kombination aus Kupfer(II)hydroxid und Kupfer (II)chlorid die Verbindung der Formel $Cu_2(OH)_2Cl_2$ oder andere Atakamite einzusetzen. Entsprechend wäre auch der Einsatz von $\alpha$- bzw. $\beta$-$Fe_2(OH)_3Cl$ denkbar.

In den Kombinationen b) liegen die Einzelkomponenten ba) und bb) in solchen Mengenverhältnissen vor, daß auf jedes Metallatom der Komponente b) (ba und gegebenenfalls (i)) 0,1 bis 2, vorzugsweise 0,5 bis 1,5 Chloratome der Komponente bb) (i) und (ii)) entfallen. Der Gewichtsanteil der Cokatalysatorkomponente ba) soll hierbei vorzugsweise mindestens ein drittel und höchstens neun zehntel der gesamten Cokatalysatorkomponente b) ausmachen.

Die als Cokatalysatoren b) geeigneten Verbindungen werden im allgemeinen insgesamt mit 0,5 bis 20 Gew.-%, vorzugsweise mit 2 bis 10 Gew.-%, bezogen auf die Gesamtmenge des Reaktionsgemisches, incl. gegebenenfalls mitverwendetem Lösungsmittel, eingesetzt.

Die Mitverwendung der Cokatalysatorkomponente b) ist wesentlich für einen hohen Anteil der Urethanbildungsreaktion gemäß Gleichung (2), d. h. nur durch die Mitverwendung der Cokatalysatorkomponente b) ist sichergestellt, daß ein hoher Anteil des in der Gasphase vorliegenden Wasserstoffs unter Hydrocarbonylierung der Nitroverbindung an der Reaktion teilnimmt. Wegen des niedrigen Chloridgehalts der Cokatalysatorkomponente b) ist das erfindungsgemäße Verfahren ohne ernsthafte Korrosionsprobleme durchführbar.

Als Cokatalysatorkomponente c) sind insbesondere ca) tertiäre organische Amine oder cb) den als Ausgangsmaterialien eingesetzten Nitroverbindungen bzw. organischen Hydroxylverbindungen strukturell verwandte primäre oder sekundäre Amine.

Geeignete tertiäre Amine ca) sind solche des Molekulargewichts von 59 bis etwa 10 000, vorzugsweise 59 bis 300, von aliphatischer, cycloaliphatischer, aromatischer, araliphatischer oder heterocyclischer Art. Ebenfalls geeignet sind tert. Amine, welche unter den Reaktionsbedingungen inerte Substituenten wie beispielsweise Halogen-, Cyano-, Alkoxy-, Phenoxy-, Thiophenoxy-, Carbonyl-, Carboalkoxy- und/oder Thiocarbonyl-Substituenten aufweisen. Beispiele geeigneter tertiärer Amine sind Trimethylamin, Triethylamin, Tripropylamin, Tributylamin, cycloaliphatische tertiäre Amine, wie N,N-Dimethylcyclohexylamin, N,N-Diethylcyclohexylamin, 1,4-Diaza-[2,2,2] bicyclooctan, aromatische tertiäre Amine wie N,N-Dimethylanilin, N,N-Dimethyl-4-toluidin, N,N-Diethylanilin sowie heteroaromatische tertiäre Amine wie Pyridin, die Picoline, Chinolin, Isochinolin, Chinaldin, Lepidin, Imidazol, Pyrazol, Benzimidazol, pyrolisiertes Polyacrylnitril oder Polyvinylpyridin. Diese Amine können mit 0,5 bis etwa 5 Gew.-% im erfindungsgemäßen Verfahren verwendet werden, jeweils bezogen auf die gesamten, nicht gasförmigen Einsatzstoffe.

Bevorzugte Cokatalysatoren c) sind jedoch solche des genannten Typs cb). Ihre Struktur hängt von der Art der einzusetzenden Nitroverbindung und gegebenenfalls des verwendeten Alkohols ab : Setzt man eine organische Nitroverbindung der Formel $R^1—NO_2$ erfindungsgemäß ein, so stellen solche Amine, die sich formal durch Reduktion der Nitrogruppe zur Amingruppe und gegebenenfalls formal durch N-Alkylierung dieser Amine durch den als Ausgangsmaterial eingesetzten Alkohol $R^2—OH$ daraus ableiten lassen, den Amintyp cb) dar :

$$R^1-\overset{H}{\overset{|}{N}}-R^3$$

mit $R^1 = R^1$ aus $R^1—NO_2$ und $R^3 = H$ oder $R^2$ aus $R^2—OH$.

Enthält die eingesetzte Nitroverbindung mehr als eine Nitrogruppe, so leiten sich daraus formal Amine ab, die ein- oder mehrfach die Strukturmerkmale des Amintyps cb) aufweisen und ebenfalls erfindungsgemäße Cokatalysatoren c) darstellen. Gegebenenfalls in der Aminverbindung vorhandene Nitrogruppen reagieren dann jedoch erfindungsgemäß zu den entsprechenden Urethanen ab.

Die Amine des Typs cb) können bis zu etwa 15 Gew.-%, bezogen auf das gesamte Einsatzgemisch incl. Lösungsmittel, im erfindungsgemäßen Verfahren eingesetzt werden. Vorzugsweise werden sie in einer Menge verwendet, die molar ein zwanzigstel bis ein drittel der eingesetzten Nitroverbindung ausmacht. In diesem Zusammenhang muß jedoch darauf aufmerksam gemacht werden, daß gegebenenfalls als Cokatalysator cb) eingesetzte primäre Amine selbst bei deren Einsatz in relativ hohen Mengen ausschließlich die Funktion eines Cokatalysators ausüben, da sie im Gegensatz zur Lehre der oben zitierten japanischen Veröffentlichung 57-185253 nicht im Sinne eines erfindungsgemäß einzusetzenden Ausgangsmaterials zu Urethanen abreagieren.

Das Katalysatorsystem des erfindungsgemäßen Verfahrens kann durch Zusätze von feinverteiltem metallischem Eisen oder Nickel ergänzt werden. Diese Metallzusätze wirken reaktionsbeschleunigend ; wenn chloridhaltige Cokatalysatoren zugegen sind, wirkt ein Eisenzusatz auch korrosionsinhibierend. Im allgemeinen werden bis etwa 3 Gew.-%, bezogen auf das gesamte Reaktionsgemisch, incl. evtl. mitverwendetem Lösungsmittel, an metallischem Eisen bzw. Nickel zugefügt.

Erfindungswesentlich ist neben dem speziellen Cokatalysatorsystem die Verwendung eines Gemischs aus Kohlenmonoxid und Wasserstoff anstelle von reinem Kohlenmonoxid. Geeignete Einsatzgase enthalten Kohlenmonoxid und Wasserstoff im Molverhältnis bzw. Volumenverhältnis 0,3 : 1 bis 3 : 1 und vorzugsweise 0,5 : 1 bis 1,5 : 1. Das Einsatzgas kann neben den Hauptkomponenten Kohlenoxid und Wasserstoff je nach Herkunft noch weitere Bestandteile wie z. B. Helium, Argon, Stickstoff, Methan und/oder Kohlendioxid enthalten, die sich unter den Reaktionsbedingungen des Verfahrens praktisch inert verhalten und die Umsetzung nicht stören.

Das Einsatzgas für das erfindungsgemäße Verfahren kann an sich beliebig hergestellt werden, beispielsweise auch durch Mischen der Einzelkomponenten CO und $H_2$. Die wirtschaftlichen Vorteile des erfindungsgemäßen Urethanverfahrens kommen jedoch nur im Verbund mit einer geeigneten Gasversorgung bzw. Gaserzeugung zum Tragen, die ein Einsatzgas in den oben angegebenen $CO/H_2$-Verhältnissen gegebenenfalls nach einer Reinigungsstufe vorzugsweise schwefelfrei, wie z. B. auch für die Oxosynthese gefordert, liefern. Da beim erfindungsgemäßen Verfahren im wesentlichen aus Kohlenmonoxid und Wasserstoff bestehende Synthesegase in der gleichen Zusammensetzung eingesetzt werden können, wie sie bei den üblichen Verfahren zur Herstellung von Synthesegas anfallen und in dieser Zusammensetzung abreagieren, ermöglicht das erfindungsgemäße Verfahren in einer integrierten Gas- und Urethanproduktionsanlage die Verwendung einer kleiner dimensionierten Gaserzeugungsanlage als dies bei den bekannten Verfahren unter alleiniger Verwendung von Kohlenmonoxid möglich wäre, da bei diesen Verfahren eine ca. 3-fach höhere Kohlenmonoxidmenge eingesetzt werden muß, was eine entsprechend größer dimensionierte Gaserzeugungsanlage erforderlich macht, und wobei außerdem der gleichzeitig anfallende Wasserstoff nicht verwendet werden kann.

Geeignete Gaserzeugungsverfahren werden vielfach betrieben und sind beispielsweise in « Ullmanns Enzyklopädie der technischen Chemie », Band 14, S. 357-474 (Weinheim, New York 1977) und in Winnacker, Küchler, « Chemische Technologie », Band 5, S. 260-272 und S. 422-450 (München, Wien 1981) sowie in Kirk-Othmer, « Encyclopedia of Chémical Technology », Vol. 10 pp, S. 353-442 (New York 1966) beschrieben.

Aus obiger Gleichung (3) ist ersichtlich, daß der max. Wasserstoffbedarf des erfindungsgemäßen Verfahrens mit x = 1, d. h. mit 2 Mol $H_2$ pro Mol CO und pro Mol erzeugter Urethangruppen erreicht ist, was einem $CO/H_2$-Verhältnis von 1 : 2 im Einsatzgas entspricht. Wenn ein Gas mit einem höheren Wasserstoffanteil, z. B. aus einem Erdgas-Reformer, angeliefert wird, können wahlweise die Maßnahmen 1)-3) ergriffen werden :

1) das angelieferte wasserstoffreiche Gas kann durch ein billiges Trennverfahren, z. B. das Monsanto-Membran-Trennverfahren oder durch das pressureswing-Verfahren von Wasserstoff auf den gewünschten Anteil abgereichert werden ; der abgetrennte Wasserstoff kann dann einer beliebigen anderen Verwendung zugeführt werden ;

2) man führt die Reaktion so — was mit diesem Katalysatorsystem möglich ist — daß neben der Urethanherstellung gleichzeitig die Nitroverbindung zum entsprechenden Amin reduziert wird. Das Amin aus dieser Koppelproduktion kann dann z. B. zur Farbstoffherstellung dienen ;

3) man setzt das wasserstoffreiche Gas zur Urethanherstellung ein und verwendet das durchgesetzte, wasserstoffhaltige Gas als Heizgas, beispielsweise zur Reformerunterfeuerung.

Die für das erfindungsgemäße Verfahren benötigte Synthesegasmenge ist in ihrer Untergrenze durch die Stöchiometrie gegeben, wie sie am Beispiel Phenylurethan in Gleichung (3) formuliert ist. Jedoch ist es im allgemeinen für die Umsetzung vorteilhaft, bei « geradem Durchgang » die 1,05- bis 20-fache, vorzugsweise die 1,05- bis 10-fache Menge der Stöchiometrie anzuwenden.

Gegenüber dem « geraden Durchgang » ist jedoch eine « Kreisgasfahrweise » bevorzugt. Zur Durchführung des Verfahrens mit Kreisgas wird das den letzten Reaktor verlassende Reaktionsgas über einen Kompressor zum Eingang der Reaktionsstufe zurückgeführt, mit frischem Einsatzgas vermischt und in die Reaktionsstufe eingespeist. Das bei der Urethanherstellung gebildete $CO_2$ wird im Kreisgassystem über eine geeignete Trennstufe ausgeschleust. Mit Ausnahme von $CO_2$ sollten die Inertgasbestandteile im Einsatzgas möglichst gering sein, um ein Aufkonzentrieren von Inertgas im Kreisgas zu verhindern.

Die Kreisgasfahrweise bietet mehrere Vorteile :

1) Das Einsatzgas wird optimal genutzt, da abgesehen von geringen Verlusten für die $CO_2$-Ausschleusung, nur der stöchiometrische Gasbedarf bereitzustellen ist.

2) Die Enthalpie der stark exothermen Urethanbildungsreaktion kann ganz oder teilweise über einen Kühler bzw. Kondensator im Kreisgassystem abgeführt werden ; die Wärmeabfuhr über Wärmetauscher in dem feststoffhaltigen Reaktionsmedium ist technisch schwieriger.

3) Die Konzentration der gasförmigen Reaktionsteilnehmer Kohlenmonoxid und Wasserstoff kann den Erfordernissen der kinetischen Reaktionsführung angepaßt werden und ist weitgehend unabhängig von den Konzentrationen im Einsatzgas.

Das Kreisgassystem wird vorzugsweise bei ähnlichem Druck wie die Reaktionsstufe betrieben. Das Volumen-Verhältnis des Kreisgases zu dem frischen Einsatzgas wird in dem Bereich 1 : 1 bis 100 : 1, vorzugsweise 1 : 1 bis etwa 30 : 1 gewählt. Die Ausschleusung des produzierten $CO_2$ und des evtl. im Einsatzgas vorhandenen $CO_2$ aus dem Kreisgas erfolgt nach bekannten Verfahren, wie Membrantrennverfahren oder Absorptionsverfahren. Im Falle der $CO_2$-Absorption ist es günstig, eine Absorptionsflüssigkeit oder ein Gemisch aus Flüssigkeiten zu verwenden, welche ohnehin in der Reaktionsstufe der Urethanherstellung zum Einsatz kommen, da so das Einschleppen von Fremdkomponenten vermieden wird. Beispielsweise können Ethanol, Nitrobenzol, Anilin und N-Ethylanilin oder Gemische davon als Absorptionsflüssigkeiten für $CO_2$ verwendet werden, falls N-Phenyl-O-ethylurethan produziert wird. Bei hohem $CO_2$-Partialdruck im Kreisgassystem ist die $CO_2$-Abtrennung durch Kondensation besonders wirtschaftlich und wird in diesem Fall besonders bevorzugt.

Muß $CO_2$ aus dem Kreisgas ausgeschleust werden (x in Gleichung (3) kleiner als 1, so ist dies einfacher durchzuführen, wenn ein hoher Grundpegel von $CO_2$ vorhanden ist, als wenn $CO_2$ jeweils auf einen geringen Restgehalt nahe 0 % entfernt werden muß. Da $CO_2$ die Reaktionsführung selbst in größeren Anteilen überraschenderweise nicht stört, wird vorzugsweise eine $CO_2$-Konzentration von bis zu 70 Vol.-%, bevorzugt von 20 bis 60 Vol.-%, im Kreisgas als Inertgasanteil gewählt.

Die erfindungsgemäße Umsetzung kann in Gegenwart oder in Abwesenheit eines Lösungsmittels durchgeführt werden. Im allgemeinen dient die vorzugsweise im Überschuß eingesetzte organische Hydroxylverbindung als Lösungsmittel. Die Mitverwendung inerter Lösungsmittel, die bis zu 80 Gew.-% des gesamten Reaktionseinsatzes ausmachen können, ist jedoch auch möglich. Die Menge des Lösungsmittels muß, gleichgültig ob es sich um im Überschuß eingesetzte Hydroxylverbindung oder um inertes Lösungsmittel handelt, so bemessen sein, daß die Reaktionswärme der exothermen Urethanbildung ohne unzulässige Temperaturerhöhung abgeführt werden kann und im Reaktor eine

rührbare Reaktionsphase erhalten bleibt.

Brauchbare Lösungsmittel sind gegen die Reaktionskomponenten und das Katalysatorsystem inerte Lösungsmittel, wie beispielsweise aromatische, cycloaliphatische und aliphatische Kohlenwasserstoffe, die gegebenenfalls durch Halogen substituiert sein können, wie Benzol, Toluol, Xylol, Chlorbenzol, Dichlorbenzol, Trichlorbenzol, Chlornaphthalin, Cyclohexan, Methylcyclohexan, Methylenchlorid, Tetrachlorkohlenstoff, Tetrachlorethan, Trichlor-trifluorethan u. ä. Verbindungen.

Die Reaktionstemperatur liegt im allgemeinen zwischen 100 und etwa 300 °C, insbesondere zwischen 130 und 250 °C und besonders vorteilhaft im Bereich von 140 bis 220 °C. Der Druck muß so bemessen sein, daß stets das Vorliegen einer flüssigen Phase gewährleistet ist und liegt im allgemeinen im Bereich von 5 bis 500 bar, besonders vorteilhaft im Bereich von 30 bis 300 bar bei Reaktionstemperatur. Je nach eingesetzter Nitroverbindung bzw. Hydroxyverbindung beträgt die für quantitativen Umsatz benötigte Reaktionszeit wzischen einigen Minuten und mehreren Stunden.

Die erfindungsgemäße Urethanherstellung kann diskontinuierlich oder kontinuierlich durchgeführt werden. Die Materialkostenvorteile von Kohlenmonoxid/Wasserstoffgemischen anstelle von Kohlenmonoxid als Einsatzgas kommen jedoch erst in einer kontinuierlich betriebenen Großanlage im Verbund mit einer geeigneten Gasversorgung zum tragen, weshalb die kontinuierliche Prozeßführung bevorzugt wird.

Die diskontinuierliche Urethanherstellung wird in einem Reaktor durchgeführt, die kontinuierliche Urethanherstellung im allgemeinen in einer für den verfahrensgemäßen Druckbereich ausgelegten Reaktorkaskade mit 2 bis etwa 8 Reaktoren, vorzugsweise mit 3 bis 5 Reaktoren. Da die meisten der erfindungsgemäßen Katalysatorsysteme nicht-lösliche Feststoffe enthalten bzw. bei der Reaktion solche bilden, muß das feststoffhaltige Reaktionsgemisch (Suspension) kräftig gerührt werden. Statt des Rührens kann die Suspension auch durch kräftiges Umpumpen, z. B. in Rührkesseln mit Schlaufe oder in Schlaufenreaktoren bewegt werden, wobei in der Schlaufe vorteilhaft evtl. benötigte Wärmeaustauscher angebracht sind. Bei kontinuierlicher Reaktionsführung ist es auch möglich, durch Eindüsen der Einsatzstoffe und der Reaktionsgase eine ausreichende Umwälzung der Reaktionssuspension zu gewährleisten, beispielsweise in einer Kaskade von Strahldüsenreaktoren.

Die flüssigen Einsatzstoffe des erfindungsgemäßen Verfahrens einschließlich der flüssigen Katalysatorkomponenten der Amine c) können der Reaktion einzeln oder im Gemisch zugeführt werden. Es ist jedoch vorteilhaft, die löslichen Katalysatorbestandteile in den flüssigen Einsatzstoffen gelöst einzufahren und evtl. vorhandene feste Katalysatorbestandteile, z. B. α-$Fe_2O_3$, in einem Teil oder in der gesamten Einsatzlösung suspendiert in die Reaktionsstufe einzubringen.

Eine Reaktorkaskade mit mehr als zwei Reaktoren wird für die erfindungsgemäße Durchführung des Verfahrens bevorzugt, da sie eine Optimierung der Urethanausbeute gestattet, indem wahlweise dem ersten Reaktor- nur ein Drittel bis etwa zwei Drittel der umzusetzenden Nitroverbindung und die verbleibende Menge der Nitroverbindung einem oder mehreren der nachfolgenden Reaktoren — mit Ausnahme des letzten — zugeführt wird und/oder indem die Reaktoren mit abgestuften Temperaturen betrieben werden.

Das Einsatzgas bzw. das Reaktionsgas kann bezüglich der Reaktionssuspension im Gleichstrom, im Kreuzstrom oder im Gegenstrom gefahren werden, vorzugsweise aber im Gleichstrom.

Bei gleichzeitig hoher Hydrieraktivität des urethanbildenden Katalysatorsystems oder bei hohem Wasserstoffpartialdruck im Reaktionsbereich können neben dem Urethan weitere Produkte erhalten werden. Mit den Einsatzstoffen Nitrobenzol und Ethanol können beispielsweise auch Anilin, N-Ethylanilin, N,N-Diethylanilin, N,N'-Diphenylharnstoff, Chinaldin und Triphenylisocyanurat entstehen. Die Bildung dieser Nebenprodukte kann, ohne hier eine bestimmte Reaktionsweise festzulegen, mit den Gleichungen (4) bis (9) beschrieben werden :

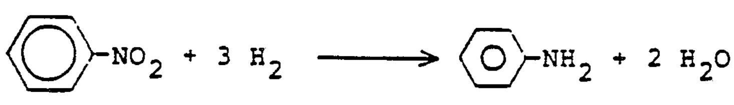
(4)

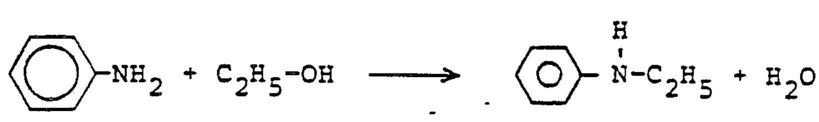
(5)

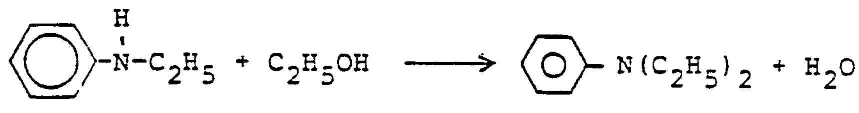
(6)

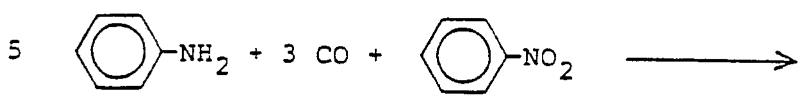
(7)

(Fortsetzung)

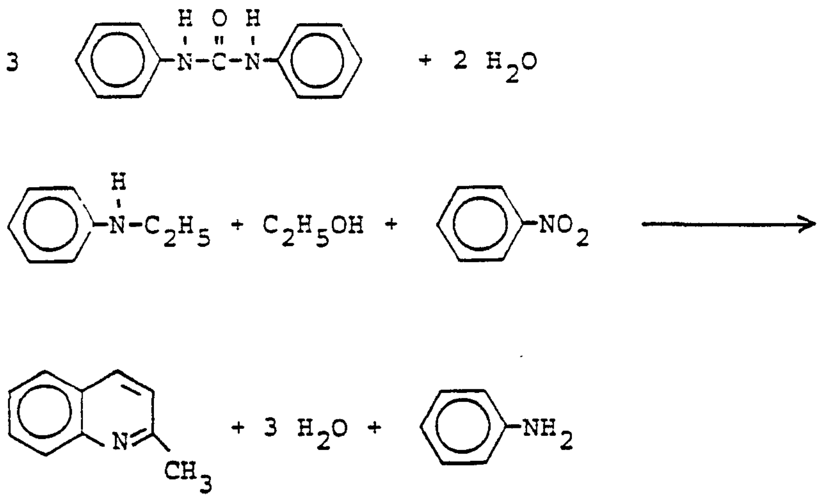

(8)

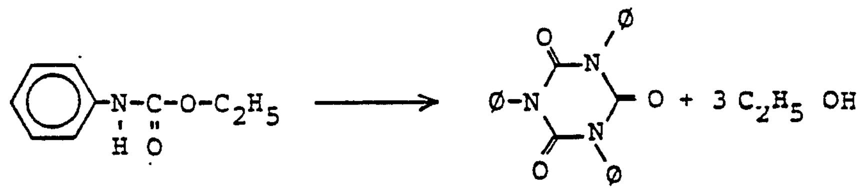

(9)

Die Nebenprodukte N,N-Diethylanilin, Chinaldin und Triphenylisocyanurat können zusammen bis etwa 4 Mol-% (bezogen auf das eingesetzte Nitrobenzol) ausmachen, meist weniger als 2 %. Da Chinaldin und N,N'-Diethylanilin auch erfindungsgemäße Katalysatorbasen ca) darstellen, können die produzierten Mengen verwendet werden, um Basenverluste zu ersetzen.

Es ist ersichtlich, daß die nach den Gleichungen (4) bis (7) gebildeten Produkte Hydrierungsprodukte der Nitroverbindung (hier Nitrobenzol) darstellen bzw. sich von derartigen Hydrierungsprodukten ableiten. Treten diese in zu hohen unerwünschten Konzentrationen auf, so können sie durch Maßnahmen, die die Nitrobenzolhydrierung beeinträchtigen, zurückgedrängt werden. Zum Beispiel kann der Wasserstoffpartialdruck in den Reaktoren durch Einstellen anderer Kreisgaskonzentrationen gesenkt werden und/oder die einzusetzende Nitroverbindung wird in einer Reaktorkaskade, wie zuvor beschrieben, einzelnen Reaktoren in Teilströmen zudosiert. Auch geringfügige Änderungen des Cokatalysatorsystems können die Selektivität der Urethanbildung erhöhen, insbesondere wenn die Konzentration der Cokatalysatorkomponente ba) erhöht wird.

Andererseits ist es im allgemeinen auch möglich, die Nebenprodukte zusammen mit den Einsatzprodukten des erfindungsgemäßen Verfahrens wieder einzusetzen, d. h. zurückzuführen. Im allgemeinen stellen sich nach mehrfacher Kreisführung die Konzentrationen der Nebenprodukte etwa gleichbleibend ein, d. h. es wird ein stationärer Zustand erreicht.

In diesem Fall treten die Nebenprodukte nur noch als Bestandteile eines flüssigen Kreislaufstroms, aber nicht mehr als Nebenprodukte des Verfahrens, auf, d. h. die Urethanselektivität wird hierdurch nicht mehr beeinträchtigt. Abweichungen von den stationären Konzentrationen der Nebenprodukte können durch eine der vorgenannten Maßnahmen ausgeglichen, geringfügige Abweichungen können gegebenenfalls auch durch einen Purge ausgeglichen werden.

Bei der Reaktionsführung, insbesondere bei der Wahl der Menge der einzusetzenden Nitroverbindung und der Lösungsmittelmenge bzw. der Überschußmenge an organischer Hydroxyverbindung ist darauf zu achten, daß bei angenommenem vollständigem Umsatz der Nitroverbindung gemäß Gleichung (3) die Wasserkonzentration in der flüssigen Phase nach der Umsetzung 10 Gew.-%, vorzugsweise 7 Gew.-%, nicht überschreitet, da bei höheren Wasserkonzentrationen die Reaktionsgeschwindigkeit und die Urethanselektivität stark absinken. Ein Binden des Reaktionswassers durch chemische oder physikalische Methoden, z. B. durch Zugabe von Orthoestern, Ketalen, Acetalen oder Molekularsieben (Zeolithen) in der Reaktionsstufe ist zwar möglich, aber wegen der erschwerten Urethanaufarbeitung im allgemeinen unwirtschaftlich.

Die Aufarbeitung der Reaktionsgemische kann beispielsweise wie nachstehend beschrieben erfolgen. Selbstverständlich sind auch andere Methoden der Aufarbeitung möglich.

Das Reaktionsgas und die flüssige Produktlösung, in welchen feste Katalysatorbestandteile suspendiert sind, werden aus der letzten Reaktionsstufe gemeinsam entspannt, beispielsweise über einen Mehrphasenwendelrohrverdampfer und gelangen in einen Gas/Flüssigkeitsabscheider, welcher zugleich als Eindicker dient. Die Temperatur und die Druckstufe dieses Abscheiders wird vorzugsweise

so gewählt, daß eine Gasphase, welche die Reaktionsgase, Wasser und einen Teil des Lösungsmittels bzw. der organischen Hydroxyverbindung enthält und eine flüssige Phase, welche der aufkonzentrierten, feststoffhaltigen Reaktionslösung (Suspension) entspricht, anfallen. Es ist jedoch auch möglich, das Reaktionsgas und die katalysatorhaltige Reaktionslösung (Suspension) über einen Druckabscheider unter Reaktionsdruck zu trennen und nur die Reaktionslösung zu entspannen.

Die Gasphase wird nach Auskondensieren des Wassers und der organischen Bestandteile auf die gewünschte $CO_2$-Konzentration eingestellt, mit frischem Einsatzgas versetzt und in die Reaktion zurückgeführt.

Die Suspension, welche die Katalysatorbestandteile, Lösungsmittel bzw. organische Hydroxyverbindung und die Verfahrensprodukte enthält, wird (bei etwa 30 bis 80 °C) durch Filtrieren, Dekantieren oder Zentrifugieren von festen Katalysatorbestandteilen befreit. Die abgetrennte Katalysatormasse bzw. Katalysatorschlamm enthält weitgehend die Katalysatorkomponente b) und darauf niedergeschlagen in feinverteilter Form die Edelmetallkomponente a) sowie evtl. ausgefallene Komplexe der Aminbasen mit den Metallhalogeniden des Katalysatorsystems. Die Katalysatormasse wird in die Reaktionsstufe zurückgeführt, das Filtrat wird dann weiter aufgearbeitet.

Die Abtrennung der Katalysatormasse vom urethanhaltigen Filtrat kann beispielsweise in geeigneten Filtereinheiten, in Zentrifugen, in Dekantern oder Querstromfiltrationsapparaten erfolgen. Die Querstromfiltration, bei welcher die Suspension in Schlauchmembranen bis auf die Konsistenz eines gerade noch pumpbaren Katalysatorschlamms eingedickt wird, wird für die Fest/Flüssig-Trennung besonders bevorzugt.

Das Filtrat kann in geringen Mengen gelöste oder kolloide anorganische Katalysatorbestandteile enthalten. Diese werden vor der weiteren Aufarbeitung zweckmäßigerweise entfernt, beispielsweise durch einen Ionenaustauscher oder durch Ausfällung. Wird z. B. mit einem Katalysatorsystem ; welches Eisen- und Chloridionen enthält, gearbeitet, so kann die Ausfällung der noch gelösten Eisenverbindungen durch Zugabe sehr geringer Mengen einer Lauge, beispielsweise durch Natronlauge oder durch Zugabe eines Alkali- bzw. Erdalkali-alkoholats bewirkt werden. Die gefällte bzw. ausgeflockte Eisenverbindung wird dann in einer zweiten Stufe zur Fest/Flüssig-Trennung entfernt.

Das Filtrat enthält nach dieser gegebenenfalls erforderlichen Nachbehandlung die Verfahrensprodukte, evtl. nicht-umgesetzte flüssige Einsatzstoffe bzw. die flüssigen Aminbasen sowie evtl. geringe Wassermengen, welche zunächst evtl. im Azeotrop mit einem der anderen Lösungsbestandteile destillativ entfernt werden. Die weitere Auftrennung der urethanhaltigen Phase richtet sich nach der Art des Lösungsmittels bzw. der überschüssigen, Hydroxygruppen enthaltenden organischen Verbindung, der Art der Aminbasen und des Urethans und erfolgt nach an sich bekannten Verfahren durch Destillation und/oder Extraktion und/oder Kristallisation.

Die abgetrennten Aminbasen werden zurückgeführt und bilden zusammen mit dem rückgeführten Katalysatorschlamm bzw. evtl. hinzugefügtem Frischkatalysator das von neuem einzusetzende Katalysatorsystem des erfindungsgemäßen Verfahrens. Die weiteren in der Aufarbeitungsstufe angefallenen Flüssigkeitsströme, die beispielsweise Wasser und überschüssige Hydroxyverbindung und/oder inertes Lösungsmittel sowie evtl. nicht-umgesetzte Nitroverbindung enthalten, werden gleichfalls in die Reaktionsstufe zurückgeführt. Die wasserhaltigen Ströme werden zuvor nach geeigneten Verfahren, beispielsweise durch Azeotropdestillation, so weit entwässert, daß der Gesamtwassergehalt der flüssigen Phasen am Reaktoreingang im Bereich von etwa 0,1 bis 0,8 Gew.-%, vorzugsweise bei 0,1 bis 0,3 Gew.-%, liegt.

Die erfindungsgemäßen Verfahrensprodukte (Urethane) eignen sich als Schädlingsbekämpfungsmittel bzw. als Zwischenprodukte zur Herstellung von Schädlingsbekämpfungsmitteln. Sie sind jedoch in erster Linie als Ausgangsmaterialien zur Herstellung der ihnen zugrundeliegenden Isocyanaten von Interesse. Diese Herstellung der Isocyanate erfolgt durch an sich bekannte thermische Spaltung der erfindungsgemäßen Verfahrensprodukte.

Die nachfolgenden Beispiele dienen zur weiteren Erläuterung des erfindungsgemäßen Verfahrens, ohne dieses einzuschränken.

### Beispiele 1-11, allgemeine Arbeitsweise

Die Versuche wurden in einem 0,7 l Edelstahlautoklaven mit jeweils 200 g Ethanol, der im übrigen durch Gew.-% chrakterisierten Einsatzgemische durchgeführt. Der gefüllte Autoklav wurde zunächst mit Stickstoff gespült, danach wurden bei Raumtemperatur die Gase in der Reihenfolge Kohlendioxid (falls verwendet), Kohlenmonoxid und Wasserstoff mit den angegebenen Partialdrücken aufgepreßt. Das Gemisch wurde unter Rühren auf Reaktionstemperatur gebracht ; nach Ablauf der Reaktionszeit ließ man auf Raumtemperatur abkühlen. Die Gasphase wurde über eine Gasuhr in einen Gassack, aus welchem eine Gasprobe zur gaschromatographischen Analyse entnommen wurde, entspannt. Das erhaltene Reaktionsgemisch wurde ausgewogen und durch Gaschromatographie sowie Hochdruckflüssigkeitschromatographie analysiert.

Die Selektivität wird in Mol.-%, bezogen auf umgesetztes Nitrobenzol, angegeben. Falls im Einsatzgemisch Amine vorhanden sind, die auch als Reaktionsprodukte auftreten können, so werden für die Selektivitätsberechnung die eingesetzten Aminmengen von den gefundenen vorab substrahiert. Für

Phenylurethan wird eine Selektivität $S_I$ angegeben, die dem unmittelbar aus Nitrobenzol erhaltenen Phenylurethan entspricht. Eine weitere Selektivität für Phenylurethan $S_{II}$, wird aus der Summe der Selektivitäten von Anilin, N-Ethylanilin, N,N'-Diphenylharnstoff und $S_I$ errechnet und gibt die erreichbare Gesamtselektivität für Phenylurethan bei Rückführung der Nebenprodukte an.

Der Hydrocarbonylierungsgrad HCG wird in % angegeben und entspricht dem 100-fachen des in Gleichung (3) definierten Wertes x. Der HCG beschreibt den Anteil der nach Gleichung (2) gebildeten Urethanmenge, bezogen auf das gesamte gebildete Phenylurethan. Der HCG-Wert wird aus der entstandenen Wassermenge errechnet ; die Wasseranteile, die auf Hydrierreaktionen bzw. Kondensationsreaktionen gemäß den Gleichungen (4) bis (8) entfallen, werden nach diesen Gleichungen bestimmt und für die Ermittlung des HCG ausgeschieden. Ein Kontrollwert für den HCG kann aus der $CO_2$-Analyse des entspannten Reaktionsgases ermittelt werden. Da $CO_2$ aber auch in den flüssigen Produktlösungen gelöst verbleibt und analytisch nicht erfaßt wird, liefert diese Methode regelmäßig einen etwas zu hohen Wert für den HCG.

## Beispiel 1

Ein Gemisch aus 73,2 % Ethanol, 18,3 % Nitrobenzol, 3,7 % Anilin, 2,9 % $\alpha$-$Fe_2O_3$, 1,8 % $VOCl_3$ und 80 ppm $PdCl_2$ wurde mit 60 bar CO und 60 bar Wasserstoff 1 Stunde bei 180 °C umgesetzt. Der Nitrobenzolumsatz betrug 84 %, die Produktselektivitäten waren 0,5 % Anilin, 7,0 % N-Ethylanilin, 0,6 % N,N-Diethylanilin, 1,9 % Chinaldin, 8,7 % N,N'-Diphenylharnstoff und $S_I$ = 81,3 % bzw. $S_{II}$ = 97,5 % für Phenylurethan, der HCG war 62 %.

## Beispiel 2

Ein Gemisch aus 75,15 % Ethanol, 18,8 % Nitrobenzol, 3,8 % Anilin, 0,75 % CuO, 0,75 % $CuCl_2$, 0,75 % Pyridinhydrochlorid und 80 ppm $PdCl_2$ wurde mit 60 bar CO und 60 bar Wasserstoff 2 Stunden bei 180 °C umgesetzt. Der Nitrobenzolumsatz betrug 83 %, die Produktselektivitäten waren 19,4 % Anilin, 10,9 % N-Ethylanilin, 2,4 % Chinaldin, 11,1 % N,N'-Diphenylharnstoff und $S_I$ = 54,7 % bzw. $S_{II}$ = 96,1 % für Phenylurethan, der HCG war 74 %.

## Beispiel 3

Ein Gemisch aus 66,2 % Ethanol, 26,1 % Nitrobenzol, 1,6 % Anilin, 0,3 % N-Ethylanilin, 0,9 % Chinaldin, 0,3 % N,N'-Diphenylharnstoff, 0,3 % $MnCl_2$, 1,0 % $FeCl_2 \cdot 2H_2O$, 3,3 % $\alpha$-$Fe_2O_3$ und 296 ppm $PdCl_2$ wurde mit 80 bar CO und 40 bar $H_2$ 2 Stunden bei 180 °C umgesetzt. Der Nitrobenzolumsatz betrug 100 %, die Produktselektivitäten waren 4,8 % Anilin, 0,1 % N-Ethylanilin, 2,3 % N,N'-Diphenylharnstoff und $S_I$ 92,0 % bzw. $S_{II}$ 99,2 % für Phenylurethan bei 61 % HCG.

## Beispiel 4

Ein Gemisch aus 65,7 % Ethanol, 16,0 % Nitrobenzol, 6,4 % Anilin, 3,2 % N,N-Diethylanilin, 6,6 % $\alpha$-$Fe_2O_3$, 2,0 % $FeCl_2 \cdot 2H_2O$ und 100 ppm Rutheniumchlorid wurden mit 60 bar CO und 60 bar Wasserstoff 2 Stunden bei 180 °C umgesetzt. Der Nitrobenzolumsatz betrug 51 %, die Produktselektivitäten waren 17,0 % Anilin, 9,5 % N-Ethylanilin, 2,4 % Chinaldin, 10,0 % N,N'-Diphenylharnstoff und $S_I$ = 58,6 % bzw. $S_{II}$ = 95,1 % für Phenylurethan, der HCG war 51 %.

## Beispiel 5

Ein Gemisch aus 70,6 % Ethanol, 16,0 % Nitrobenzol, 3,2 % Anilin, 1,6 % N,N-Diethylanilin, 6,6 % $\alpha$-$Fe_2O_3$, 2,0 % $FeCl_2 \cdot 2H_2O$ und 100 ppm Palladiumchlorid wurden mit 60 bar CO und 60 bar Wasserstoff 1 Stunde bei 180 °C umgesetzt. Der Nitrobenzolumsatz betrug 99,4 %, die Produktselektivitäten waren 12,0 % Anilin, 5,0 % N-Ethylanilin, 1,8 % Chinaldin, 2,4 % N,N'-Diphenylharnstoff und $S_I$ = 75,6 % bzw. $S_{II}$ = 95,0 % für Phenylurethan ; der HCG war 43 %.

## Beispiel 6

Ein Gemisch aus 65,7 % Ethanol, 16,0 % Nitrobenzol, 6,4 % Anilin, 3,2 % N,N-Diethylanilin, 6,6 % $\alpha$-$Fe_2O_3$, 2,0 % $FeCl_2 \cdot 2H_2O$ und 100 ppm $PdCl_2$ wurden mit 60 bar CO und 60 bar Wasserstoff 1 Stunde bei 180 °C umgesetzt. Der Nitrobenzolumsatz betrug 99,2 %, die Produktselektivitäten waren 0 % Anilin, 3,9 % N-Ethylanilin, 0,5 % Chinaldin, 2,8 % N,N'-Diphenylharnstoff und $S_I$ = 92,6 % bzw. $S_{II}$ = 99,3 % für Phenylurethan, der HCG war 52 %.

## Beispiel 7

Ein Gemisch aus 67,2 % Ethanol, 26,5 % Nitrobenzol, 1,6 % Anilin, 0,3 % $MnCl_2$, 3,4 % $\alpha$-$Fe_2O_3$,

1,0 % $FeCl_2 \cdot 2H_2O$ und 300 ppm $PdCl_2$ wurde mit 80 bar CO und 40 bar $H_2$ 1 Stunde bei 180 °C umgesetzt. Der Nitrobenzolumsatz betrug 72 %, die Produktselektivitäten waren 0 % Anilin, 1,1 % N-Ethylanilin, 0,3 % Chinaldin, 0,2 % N,N'-Diphenylharnstoff und $S_I$ =98,0 % bzw. $S_{II}$ =99,3 % für Phenylurethan, der HCG war 64 %. Dieses Beispiel belegt, daß selbst bei niedrigem Wasserstoffpartialdruck im Reaktionsgas bzw. einem $CO/H_2$-Verhältnis von 2/1 ein hoher HCG erreicht werden kann. Für die Übertragung auf kontinuierliche Reaktionsführung mit einem Kreisgassystem kann dieses Ergebnis so interpretiert werden, daß das $CO/H_2$-Verhältnis im Einsatzgas möglichst dem $CO/H_2$-Verhältnis, welcher sich als $CO/H_2$-Verbrauch nach Gleichung (3) ergibt, entsprechen soll, daß das $CO/H_2$-Verhältnis im Reaktionsgas bzw. im Kreisgas jedoch den Erfordernissen der kinetischen Reaktionsführung, d. h. dem gewünschten HCG, angepaßt werden muß.

Beispiel 8

Ein Gemisch aus 73,0 % Ethanol, 16,6 % Nitrobenzol, 3,3 % Anilin, 1,7 % N,N-Diethylanilin, 3,3 % α-$Fe_2O_3$, 2,1 % $FeCl_2 \cdot 2H_2O$ und 100 ppm $PdCl_2$ wurde in Gegenwart von 40 bar $CO_2$ mit 40 bar CO und 80 bar $H_2$ 1 Stunde bei 180 °C umgesetzt. Der Nitrobenzolumsatz betrug 99,5 %, die Produktselektivitäten waren 55,0 % Anilin, 3,7 % N-Ethylanilin, 0,4 % Chinaldin, 2,4 % N,N'-Diphenylharnstoff und $S_I$ =39 % bzw. $S_{II}$ = 99,6 % für Phenylurethan, der HCG war 32 %.

Beispiel 9

Ein Gemisch aus 69,2 % Ethanol, 15,7 % Nitrobenzol, 3,1 % Anilin, 1,6 % N,N-Diethylanilin, 6,5 % α-$Fe_2O_3$, 3,9 % $FeCl_2 \cdot 2H_2O$ und 100 ppm $PdCl_2$ wird in Gegenwart von 40 bar $CO_2$ mit 40 bar CO und 80 bar $H_2$ 1 Stunde bei 180 °C umgesetzt. Der Nitrobenzolumsatz betrug 99,9 %, die Produktselektivitäten waren 27,1 % Anilin, 4,0 % N-Ethylanilin, 2,9 % N,N'-Diphenylharnstoff und $S_I$ =65,4 % bzw. $S_{II}$ = 99,4 % für Phenylurethan, der HCG war 61 %. Dieses Beispiel beweist im Vergleich zu Beispiel 8, daß eine Konzentrationserhöhung der eisenhaltigen Cokatalysatorkomponenten selbst bei hohem Wasserstoffpartialdruck die Hydrierprodukte zugunsten der Hydrocarbonylierungsreaktion zurückdrängt.

Beispiel 10 (Vergleichsbeispiel)

Ein Gemisch aus 83,3 % Ethanol, 10,2 % Nitrobenzol, 1,6 % Anilin, 1,5 % 1,4-Diaza-2,2,2-bicyclooctan und 0,4 % metallischem Selen wurde mit 60 bar CO und 60 bar Wasserstoff 1 Stunde bei 180 °C umgesettz. Der Nitrobenzolumsatz betrug 86 %, die Produktselektivitäten waren 0,2 % Chinaldin, 5,4 % N,N'-Diphenylharnstoff und $S_I$ = 89,7 % bzw. 95,1 % für Phenylurethan, der HCG war 1,9 %.

Beispiel 11 (Vergleichsbeispiel)

Es wurde wie in Beispiel 10, jedoch zusätzlich mit 600 ppm Palladiumchlorid im Einsatz, gearbeitet. Der Nitrobenzolumsatz betrug 99,5 %, die Produktselektivitäten waren 0,4 % Chinaldin, 4,4 % N,N'-Diphenylharnstoff und $S_I$ = 92,8 % bzw. $S_{II}$ = 97,2 % für Phenylurethan, der HCG war 0 %.

Die Beispiele 10 und 11 beweisen als Vergleichsbeispiele, daß mit Se bzw. Se und Pd enthaltenden Katalysatorsystemen zwar auch mit $CO/H_2$-Gemischen hohe Urethanausbeuten erhalten werden, daß jedoch eine Reaktion gemäß Gleichung (2) — wie aus dem HCG ersichtlich — nicht oder nur ganz untergeordnet stattfindet, d. h. daß die Urethanbildung praktisch ohne Wasserstoffverbrauch abläuft.

Beispiel 12

In einem 0,7 l Edelstahlautoklaven wurde ein Gemisch aus 200 g Ethanol, 20 g 2,4-Dinitrotoluol, 5 g Pyridin, 4 g 2,4-Diaminotoluol, 15 g α-$Fe_2O_3$, 5 g $FeCl_2 \cdot 2H_2O$ und 25 mg Palladiumchlorid mit 60 bar CO und 60 bar Wasserstoff versetzt und 1 Stunde bei 180 °C zur Reaktion gebracht. Nach dem Abkühlen wurde der Autoklav geöffnet, zum Autoklaveninhalt wurden 10 g 2,4-Dinitrotoluol hinzugefügt, und es wurde nochmals in der gleichen Weise mit frischem $CO/H_2$-Gemisch umgesetzt. Die Produktanalyse aus der Hochdruckflüssigkeitschromatographie zeige 100 % Umsatz des 2,4-Dinitrotoluols an ; die Selektivitäten waren 14,5 % für Monourethane und 43 % für Toluol-2,4-dicarbamat, das gewünschte Bisurethan. Daneben wurden harnstoffartige Produkte nachgewiesen. Der HCG wurde zu 45 % errechnet. Die Nebenprodukte sind rückführbar und liefern das gewünschte Bisurethan.

Beispiele 13-16, allgemeine Arbeitsweise

Zur kontinuierlichen Versuchsdurchführung wurde eine Reaktorkaskade aus zwei einfach verbundenen 2,5 l Rührkesseln (Hastelloy C) mit je 2,2 l Füllhöhe aufgebaut. Der 1. Kessel wurde über einen Durchflußregler mit einem konstanten Gasstrom von CO und $H_2$ und über eine Kolbenpumpe aus einer kräftig gerührten Vorlage mit der Suspension, welche alle nicht gasförmigen Einsatzstoffe enthielt, beschickt. Falls auch $CO_2$ eingesetzt wurde, so wurde dieses separat flüssig dosiert, in einer beheizten

Zuleitung verdampft und vor dem Reaktor dem übrigen Gasstrom zugemischt. Die Reaktoren wurden elektrisch beheizt und über Magnetkupplung gerührt. Der Reaktionsdruck wurde über ein Regelventil, über welches die gesamte Reaktionsmasse entspannt wurde, am Ausgang des zweiten Reaktors gehalten. Alle Suspension führenden Leitungen im Druckteil und nach dem Entspannungsventil waren auf ca. 100 °C beheizt. Die Produkte wurden in einem Glasabscheider in eine Suspension und in eine Gasphase getrennt, die einzelnen Phasen wurden bilanziert und analysiert. Die Analysen- und Auswertungsmethode entsprach der für die Beispiele 1 bis 11 angegebenen. Ein Versuch dauerte jeweils 24 Stunden, die Versuchsergebnisse sind aus jeweils drei Analysen, d. h. aus Proben in 8-stündigen Intervallen gemittelt.

Beispiel 13

Einsätze (Gew.-%) : Ethanol (70,6), Nitrobenzol (16,0), Anilin (3,2), N,N-Diethylanilin (1,6), α-$Fe_2O_3$ (6,6), $FeCl_2 \times 2H_2O$ (2,0) und 110 ppm $PdCl_2$

| | | |
|---|---|---|
| Durchsatz der Suspension: | 2000 g/h | |
| CO-Durchsatz: | 588 g/h | Gaszusammensetzung am Reaktoreingang: 50 Vol.-% CO, 50 Vol.-% $H_2$. |
| $H_2$-Durchsatz: | 42 g/h | |
| Reaktionsdruck: | 90 bar | |
| Reaktionstemperatur: | 180°C | |

Der Nitrobenzolumsatz betrug 100 %, die Selektivität der rückführbaren Nebenprodukte war 17 %, $S_I$ = 79 % bzw. $S_{II}$ = 96 % für Phenylurethan bei 68 %HCG.

Beispiel 14

Es wurde wie in Beispiel 13 gearbeitet, jedoch wurden zusätzlich 1 320 g/h $CO_2$ eingefahren, womit sich am Reaktoreingang eine Gaszusammensetzung von 42 Vol.-% $CO_2$, 29 Vol.-% CO und 29 Vol.-% $H_2$ ergab.

Der Nitrobenzolumsatz war 97 % bis 100 %, die Selektivität der rückführbaren Nebenprodukte war 19 %, $S_I$ = 75 % bzw. $S_{II}$ = 94 % für Phenylurethan bei 61 % HCG.

Beispiel 15

Einsätze (Gew.-%) : Ethanol (77,3), Nitrobenzol (16,0), Anilin (1,6), N,N-Diethylanilin (0,8), α-$Fe_2O_3$ (3,3), $FeCl_2 \cdot 2H_2O$ (1,0) und 110 ppm $PdCl_2$

| | | |
|---|---|---|
| Durchsatz der Suspension: | 2000 g/h | |
| CO-Durchsatz: | 588 g/h | Gaszusammensetzung am Reaktoreingang: 29 Vol.-% CO, 42 Vol.-% $H_2$ und 29 Vol.-% $CO_2$ |
| $H_2$-Durchsatz: | 60 g/h | |
| $CO_2$-Durchsatz: | 924 g/h | |
| Reaktionsdruck: | 90 bar | |
| Reaktionstemperatur: | 180°C | |

Der Nitrobenzolumsatz betrug 90 %, die Selektivität der rückführbaren Nebenprodukte war 42 %, $S_I$ = 55 % bzw. $S_{II}$ = 97 % für Phenylurethan bei 51 % HCG.

Beispiel 16

Es wurde wie in Beispiel 15, jedoch mit der vierfachen Menge Anilin und N,N-Diethylanilin im Einsatzgemisch gearbeitet.

Der Nitrobenzolumsatz betrug 92 %, die Selektivität der rückführbaren Nebenprodukte war 22 %, $S_I$ = 75 % bzw. $S_{II}$ = 97 % für Phenylurethan bei 76 % HCG.

**Patentansprüche**

1. Verfahren zur Herstellung von Urethanen durch Umsetzung von organischen Nitroverbindungen mit mindestens eine Hydroxygruppe enthaltenden organischen Verbindungen und Kohlenoxid in

Gegenwart eines Selen-freien Katalysatorsystems, welches

a) mindestens ein Edelmetall oder eine Edelmetallverbindung der 8. Nebengruppe des Periodensystems der Elemente enthält, dadurch gekennzeichnet, daß man einerseits ein solches Katalysatorsystem verwendet, welches

b) mindestens einen Cokatalysator bestehend aus einer Kombination aus

ba) mindestens einer oxidischen oder hydroxidischen Verbindung des Eisens oder des Kupfers und

bb) mindestens einer, anionisch als Chlorid gebundenes Chlor enthaltenden Verbindung ausgewählt aus der Gruppe bestehend aus

(i) Chloriden oder Oxichloriden von Elementen der dritten bis fünften Hauptgruppe und/oder der ersten bis achten Nebengruppe des Periodensystems mit Ausnahme der Edelmetalle der achten Nebengruppe und

(ii) organischen Ammoniumchloriden, und

c) als weitere Cokatalysatorkomponente mindestens eine organische Stickstoffbase

enthält, und man andererseits das Kohlenoxid zusammen mit Wasserstoff unter Einhaltung eines Molverhältnisses $CO : H_2$ von 0,3 : 1 bis 3 : 1 verwendet.

2. Verfahren gemäß Anspruch 1, dadurch gekennzeichnet, daß man als Katalysatorkomponente a) Palladium und/oder Ruthenium in feinverteilter metallischer Form und/oder in Form löslicher Verbindungen einsetzt.

3. Verfahren gemäß Ansprüchen 1 und 2, dadurch gekennzeichnet, daß man als Komponente ba) Oxide und/oder Oxidhydrate des dreiwertigen Eisens verwendet.

4. Verfahren gemäß Ansprüchen 1 bis 3, dadurch gekennzeichnet, daß man als Komponente (i) Eisen-(II)-chlorid und/oder Eisen-(III)-chlorid, sowie Hydrate oder Amin-Komplexe dieser Eisenchloride verwendet.

5. Verfahren gemäß Ansprüchen 1 bis 4, dadurch gekennzeichnet, daß man die Umsetzung kontinuierlich in einer Kaskade von Reaktoren durchführt.

6. Verfahren gemäß Anspruch 5, dadurch gekennzeichnet, daß man die Umsetzung mit einem Kreisgassystem durchführt, wobei man dem den letzten Reaktor verlassenden Gasgemisch durch Absorption oder Kondensation Kohlendioxid in einer solchen Menge entzieht, daß das verbleibende Gas noch einen Kohlendíoxidgehalt von 20 bis 60 Vol.-% aufweist.

7. Verfahren gemäß Anspruch 1 bis 6, dadurch gekennzeichnet, daß man das entspannte Reaktionsgemisch durch Querstromfiltration auf die Konsistenz eines Katalysatorschlammes eindickt, diesen in die Reaktionsstufe zurückführt und das produkthaltige Filtrat in an sich bekannter Weise aufarbeitet.

**Claims**

1. Process for the production of urethanes by reacting organic nitro compounds with organic compounds containing at least one hydroxyl group and carbon oxide in the presence of a selenium-free catalyst system which

a) contains at least one noble metal or noble metal compound of the 8th sub-group of the periodic system of elements, characterised in that on the one hand a catalyst system is used which contains

b) at least one co-catalyst consisting of a combination of

ba) at least one oxidic or hydroxidic compound of iron or copper and

bb) at least one compound containing chlorine anionically bound as chloride, selected from the group consisting of

(i) chlorides or oxychlorides of elements of the third to fifth main group and/or of the first to eighth sub-group of the periodic system, with the exception of the noble metals of the eighth sub-group and

(ii) organic ammonium chlorides, and

c) at least one organic nitrogen base as a further co-catalyst component,

and on the other hand the carbon oxide is used together with hydrogen while adhering to a molar ratio of $OC : H_2$ of 0.3 : 1 to 3 : 1.

2. Process according to Claim 1, characterised in that palladium and/or ruthenium in a finely divided metallic form and/or in the form of soluble compounds are used as catalyst component a).

3. Process according to Claims 1 and 2, characterised in that oxides and/or oxide hydrates of trivalent iron are used as component ba).

4. Process according to Claims 1 to 3, characterised in that iron(II)chloride and/or iron(III)chloride, and hydrates or amine complexes of these iron chlorides are used as component (i).

5. Process according to Claims 1 to 4, characterised in that the reaction is carried out continuously in

a cascade of reactors.

6. Process according to Claim 5, characterised in that the reaction is carried out using a recycle gas system, carbon dioxide being removed from the gas mixture leaving the last reactor by absorption or condensation in such a quantity that the remaining gas still has a carbon dioxide content of 20 to 60 % by volume.

7. Process according to Claim 1 to 6, characterised in that the expanded reaction mixture is concentrated by crossflow filtration to the consistency of a catalyst sludge which is returned to the reaction stage and the product-containing filtrate is worked up in a manner known per se.

**Revendications**

1. Procédé de préparation d'uréthanes par réaction de nitro-composés organiques avec des composés organiques contenant au moins un groupe hydroxy et de l'oxide de carbone en présence d'un système catalytique exempt de sélénium,

a) contenant au moins un métal noble ou un composé d'un métal noble du huitième sous-groupe du Système Périodique des Eléments, caractérisé en ce que, d'une part, on utilise un système catalytique contenant

b) au moins un cocatalyseur constitué d'une combinaison comprenant :

ba) au moins un composé d'oxyde ou d'hydroxyde du fer ou du cuivre, et

bb) au moins un composé contenant du chlore lié par voie anionique sous forme d'un chlorure et choisi parmi le groupe comprenant :

(i) des chlorures ou des oxychlorures d'éléments du troisième au cinquième groupes principaux et/ou du premier au huitième sous-groupes du Système Périodique, à l'exception des métaux nobles du huitième sous-groupe, et

(ii) des chlorures d'ammonium organiques, et

c) comme autre composant cocatalytique, au moins une base azotée organique,

tandis que, d'autre part, on utilise l'oxide de carbone conjointement avec de l'hydrogène en maintenant un rapport molaire $CO : H_2$ de 0,3 : 1 à 3 : 1.

2. Procédé selon la revendication 1, caractérisé en ce que, comme composant catalytique a), on utilise le palladium et/ou le ruthénium sous forme métallique finement divisée et/ou sous forme de composés solubles.

3. Procédé selon les revendications 1 et 2, caractérisé en ce que, comme composant ba), on utilise des oxydes ou des oxyhydrates du fer trivalent.

4. Procédé selon les revendications 1 à 3, caractérisé en ce que, comme composant (i), on utilise le chlorure de fer (II) et/ou le chlorure de fer (III), ainsi que les hydrates ou les complexes aminés de ces chlorures de fer.

5. Procédé selon les revendications 1 à 4, caractérisé en ce qu'on effectue la réaction en continu dans une cascade de réacteurs.

6. Procédé selon la revendication 5, caractérisé en ce qu'on effectue la réaction dans un système de gaz en cycle en retirant, du mélange gazeux quittant le dernier réacteur, par absorption ou condensation, de l'anhydride carbonique en une quantité telle que le gaz restant ait encore une teneur en anhydride carbonique de 20 à 60 % en volume.

7. Procédé selon les revendications 1 à 6, caractérisé en ce que, par filtration à courants transversaux, on épaissit le mélange réactionnel détendu à la consistance d'une boue catalytique, on recycle cette dernière à l'étape réactionnelle et on traite le filtrat contenant les produits de façon connue en soi.